# EUROPEAN PATENT APPLICATION

(11) **EP 1 946 753 A1**
(43) Date of publication of application: **23.07.2008**
(21) Application number: 06822405.4
(22) Date of filing: 20.10.2006
(51) Int. Cl.: A61K 31/196, A61K 31/352, A61K 31/436, A61K 45/00, A61K 47/18, A61P 17/00, A61P 37/08

(54) **PREPARATION FOR EXTERNAL APPLICATION COMPRISING SALT OF MAST CELL DEGRANULATION INHIBITOR HAVING CARBOXYL GROUP WITH ORGANIC AMINE**

(30) Priority: 21.10.2005 JP 2005306520
(71) Applicant: Medrx Co., Ltd., Higashikagawa-shi Kagawa 769-2712 (JP)
(72) Inventor: ENDO, Mitsuru, Higashikagawa-shi, Kagawa 7692712 (JP); HAMAMOTO, Hidetoshi, Higashikagawa-shi, Kagawa 7692712 (JP); YAMASAKI, Keiko, Higashikagawa-shi, Kagawa 7692712 (JP); MATSUMURA, Sueko, Columbus, Ohio 43210 (US)
(74) Representative: Duckworth, Timothy John
(86) International application number: PCT/JP2006/321436
(87) International publication number: WO 2007/046544

(57) **Abstract**

Mast cell degranulation inhibitors having a carboxyl group have not been developed as external preparations due to the low transdermal permeability thereof. By formulating external preparations thereof, side effects on the internal organs by oral administration can be avoided. Some of the mast cell degranulation inhibitors show drastically inferior photostability, which is also one cause of suppressed development of the drug as an external preparation.

The present invention aims at improving the transdermal permeability and photostability of mast cell degranulation inhibitors by forming a salt of the mast cell degranulation inhibitor with an organic amine. Consequently, an external preparation of a mast cell degranulation inhibitor can be provided and the photostability of the mast cell degranulation inhibitor itself, and a preparation containing same can be improved.

## Description

### Technical Field

The present invention relates to an external preparation comprising a salt of a mast cell degranulation inhibitor having a carboxyl group with an organic amine, a production method of an external preparation containing the mast cell degranulation inhibitor with improved photostability, and a method of improving the photostability of the mast cell degranulation inhibitor.

### Background of the invention

Human body has an activity to eliminate a foreign substance that invaded into or came into contact with the body from the outside. This is called an immune system, which is an extremely important activity as a defense system of the human body. The main immune system is so controlled by an immunity control system as not to respond to self tissue.

However, when the mechanism of the immunity control system is broken, an excessive response inconvenient for self sometimes occurs. This response causes a harmful action on the human body such as allergy and sensitivity.

A foreign substance that induces allergy is called an allergen, and, for example, various substances such as tick, pollen, dust, food, chemicals and the like can be allergens. While the allergy expression shows individual difference, symptoms such as urticaria and atopic dermatitis when the expression site is skin, allergic rhinitis when the expression site is nose, and bronchitis when the expression site is bronchi, are expressed.

It is generally considered that allergic conditions are expressed because mast cells are degranulated due to stimulation by allergen, which causes release of an intracellular chemical mediator, and this chemical mediator causes inflammatory responses.

First, immediate responses such as angiectasis, vascular hyperpermeability, mucus secretion, nerve stimuli, airway narrowing and the like occur due to the release of histamine, leukotriene and the like. A few hours later, inflammatory cells roll on and adhere to vessel walls due to the release of cytokine and the like, are migrated to tissues and activated, whereby a delayed response occurs. That is, allergic conditions are triggered by the degranulation of mast cells.

A drug that improves and treats such allergic conditions is an antiallergic agent. The antiallergic agent acts to suppress inflammatory responses by suppressing release or action of chemical mediators such as histamine and the like.

As antiallergic agents, histamine antagonist, mast cell degranulation inhibitor, chemical substance synthesis inhibitor, antibody production inhibitor and the like are known. Of these, mast cell degranulation inhibitors enable suppression of the expression of allergic conditions during the first degranulation stage, as mentioned above.

Conventionally, steroid external preparations are generally used for allergic dermatitis such as atopic dermatitis and the like, and have achieved a high treatment effect.

However, use of steroids requires due attention, and side effects such as skin atrophy, xeroderma, infections induced by weakened immune system, photosensitivity and pigment disorder sometimes occur, depending on the kind, dose, administration frequency, long-term administration and the like of steroids. In addition, administration to infants and elderly having delicate skin requires particular care.

Therefore, mast cell degranulation inhibitors such as tranilast, sodium cromoglycate and the like are expected to replace the above-mentioned steroid, and used for dermatic diseases such as atopic dermatitis and the like.

In such allergic dermatitis, since degranulation from mast cells occurs in the affected part of dermatic diseases, direct topical administration to the affected part of dermatic diseases is preferable.

Moreover, for example, serious side effects such as hepatic dysfunction and renal dysfunction are confirmed by oral administration of drugs such as tranilast [pharmaceutical product interview form (revised on April, 2004); Sekiseed capsule, Sekiseed dry syrup (reference 1)]. To reduce such side effect, the development of an external preparation permitting direct administration to the affected part of dermatic diseases is further desired.

However, mast cell degranulation inhibitors are currently commercially available only in the dosage forms of preparations for oral administration or transmucosal administration or eye drops (e.g., reference 1).

The reason therefor is low transdermal permeability of those mast cell degranulation inhibitors.

Moreover, mast cell degranulation inhibitors include pharmaceutical agents extremely inferior in the photostability. They are not suitable for external preparations that are directly influenced by light.

For example, tranilast exemplified above is highly unstable to light (reference 1).

Thus, administration of tranilast in the form of an external preparation to the affected part of dermatic diseases may suffer from remarkable decrease in the content due to light irradiation and intracorporeal absorption of decomposed tranilast and the like, and may cause an unexpected adverse influence such as allergic reaction and the like on the living body.

To ensure stability of tranilast to light irradiation, for example, the dosage form of capsule is employed and a method of shading preservation of a tranilast preparation is considered (reference 1).

However, all of these consider the stability of preparation itself during preservation, and the stability after administration of the preparation is not disclosed at all.

For an administration mode where tranilast is not exposed to light after administration such as internal drugs and eye drops, it is sufficient to simply secure the stability of the preparation in such manner.

However, when tranilast is applied to an affected part of dermatic diseases as an external preparation such as ointment and the like, the affected part may be covered to shield the light after application of the external preparation. In some cases, such light shielding becomes extremely difficult depending on the diseased part of the skin.

With regard to tranilast, therefore, even when an external preparation is successfully provided, improvement of the photostability becomes necessary, though production of its external preparation is difficult due to its low transdermal permeability.

At present, mast cell degranulation inhibitors have not been developed as external preparations due to the low transdermal permeability thereof. However, by producing an external preparation of a mast cell degranulation inhibitor with enhanced transdermal permeability, direct administration to dermatic diseases is enabled, and serious side effects on the internal organs by oral administration can be avoided.

Some of the mast cell degranulation inhibitors show drastically inferior photostability, which is also one cause of suppressed development of the drug as an external preparation.

Accordingly, the present invention aims at providing an external preparation wherein a mast cell degranulation inhibitor has enhanced transdermal permeability, as well as a method of improving photostability of a preparation containing a mast cell degranulation inhibitor.

### Disclosure of the Invention

The present inventors have conducted intensive studies in an attempt to solve the above-mentioned problems and found that the transdermal permeability of a mast cell degranulation inhibitor can be remarkably improved by addition of an organic amine to a mast cell degranulation inhibitor having a carboxyl group, namely, by forming a salt of a mast cell degranulation inhibitor having a carboxyl group with an organic amine to give the mast cell degranulation inhibitor as an ionic liquid, which resulted in the completion of the present invention.

Moreover, they have found that the photostability of a preparation containing a mast cell degranulation inhibitor can also be enhanced by providing the mast cell degranulation inhibitor as an ionic liquid.

That is, the gist of the present invention is as shown below.
(1) An external preparation comprising a salt of a mast cell degranulation inhibitor having a carboxyl group with an organic amine,
(2) the external preparation of the above-mentioned (1), wherein the amount of the organic amine blended relative to the mast cell degranulation inhibitor is 0.5- to 10-fold amount in a molar ratio,
(3) the external preparation of the above-mentioned (1), wherein the amount of the organic amine blended relative to the mast cell degranulation inhibitor is 0.5- to 2-fold amount in a molar ratio,
(4) the external preparation of any of the above-mentioned (1)-(3), wherein the organic amine is a substituted or unsubstituted aliphatic tertiary amine,
(5) the external preparation of any of the above-mentioned (1)-(3), wherein the organic amine is an aliphatic tertiary amine substituted by a hydroxyl group,
(6) the external preparation of any of the above-mentioned (1)-(3), wherein the organic amine is selected from triisopropanolamine, triethanolamine, diethanolamine and diphenhydramine,
(7) the external preparation of any of the above-mentioned (1)-(6), wherein the mast cell degranulation inhibitor is tranilast or cromoglycic acid,
(8) the external preparation of any of the above-mentioned (1)-(3), wherein the mast cell degranulation inhibitor is tranilast and the organic amine is triisopropanolamine,
(9) a method of producing an external preparation with improved photostability, which comprises adding an organic amine to cause formation of a salt to afford the mast cell degranulation inhibitor as an ionic liquid, so as to improve photostability of a mast cell degranulation inhibitor having a carboxyl group,
(10) the production method of the above-mentioned (9), wherein the amount of the organic amine blended relative to the mast cell degranulation inhibitor is 0.5- to 10-fold amount in a molar ratio,
(11) the production method of the above-mentioned (9), wherein the amount of the organic amine blended relative to the mast cell degranulation inhibitor is 0.5- to 2-fold amount in a molar ratio,
(12) the production method of any of the above-mentioned (9)-(11), wherein the organic amine is a substituted or unsubstituted aliphatic tertiary amine,
(6) the production method of any of the above-mentioned (9)-(11), wherein the organic amine is triisopropanolamine, triethanolamine, diethanolamine or diphenhydramine,
(14) the production method of any of the above-mentioned (9)-(13), wherein the mast cell degranulation inhibitor is tranilast or cromoglycic acid,
(15) the production method of any of the above-mentioned (9)-(11), wherein the mast cell degranulation inhibitor is tranilast and the organic amine is triisopropanolamine,
(16) a method of improving photostability of a mast cell degranulation inhibitor having a carboxyl group, which comprises blending an organic amine with the mast cell degranulation inhibitor to afford the inhibitor as an ionic liquid,
(17) the method of the above-mentioned (16), wherein the amount of the organic amine blended relative to the mast cell degranulation inhibitor is 0.5- to 10-fold amount in a molar ratio,
(18) a salt of tranilast with triisopropanolamine at a molar ratio of 1:1,
(19) a salt of cromoglycic acid with triisopropanolamine at a molar ratio of 1:1,
(20) use as an external preparation of an ionic liquid comprising a salt of a mast cell degranulation inhibitor having a carboxyl group with an organic amine,
(21) use of the above-mentioned (20), wherein the organic amine is an aliphatic tertiary amine substituted by a hydroxyl group,
(22) use of the above-mentioned (20), wherein the organic amine is selected from triisopropanolamine, triethanolamine, diethanolamine and diphenhydramine,
(23) use of the above-mentioned (20) or (21), wherein the organic amine is triisopropanolamine, and
(24) use of any of the above-mentioned (20) to (23), wherein the mast cell degranulation inhibitor is tranilast or cromoglycic acid.

### Brief Description of the Drawings

Fig. 1 is a graph showing the amount of intradermal transfer of tranilast in test subject A.
Fig. 2 is a graph showing the amount of intradermal transfer of tranilast in test subject B.

### Best Mode for Carrying Out the Invention

The external preparation of the present invention relates to a preparation characterized in that it contains an ionic liquid obtained by forming a salt of a mast cell degranulation inhibitor having a carboxyl group with an organic amine.

The "mast cell degranulation inhibitor having a carboxyl group" to be used in the present invention is not particularly limited as long as it can be used as a pharmaceutical product. For example, cromoglycic acid, tranilast, amlexanox, repirinast, tazanolast, pemirolast potassium, ketotifen fumarate, oxatomide, ibudilast and the like can be mentioned. One or more kinds selected from these drugs are preferably used.

Of these, tranilast and cromoglycic acid are preferable, and tranilast is particularly preferable.

Generally, these agents are acidic.

Note that tranilast is a drug particularly desired to be in the form of an external preparation since side effects on the internal organs are much feared when administered orally. Nevertheless, tranilast is unstable to light (reference 1) and may exhibit an adverse effect inside the living body, making it more difficult to produce an external agent thereof.

However, the present invention has improved the transdermal permeability of tranilast by containing tranilast and, for example, triisopropanolamine, i.e., a salt of these two, which in turn has simultaneously enabled production of an external agent and improvement of content decrease due to light irradiation.

Since transdermal administration of tranilast has been enabled as mentioned above, the feared side effects due to the oral administration can be avoided, and it has been clarified that the efficacy of tranilast can be extremely effectively utilized.

Tranilast has a chemical name of N-(3,4-Dimethoxycinnamoyl)anthranilic acid, and is used for the treatment of bronchial asthma, allergic rhinitis, atopic dermatitis and the like for its action to suppress development of a chemical mediator that induces allergic reaction (mast cell degranulation suppressing action).

Tranilast used in the present invention to prepare an ionic liquid can also be used by itself, or can be used as a pharmacologically acceptable salt such as an alkali metal salt (e.g., sodium salt, potassium salt and the like).

As cromoglycic acid, sodium cromoglycate is preferable.

The "organic amine" to be used in the present invention is a compound having a cationizable nitrogen atom, and is not particularly limited as long as it is pharmaceutically acceptable. For example, primary amines such as ethanolamine and the like, secondary amines such as diethanolamine, diisopropanolamine and the like, tertiary amines such as triethanolamine, triisopropanolamine and the like, aniline substances, pyridine substances such as nicotinic acid amide and the like, amino acids such as alanine, arginine, glycine and the like, drugs such as diphenhydramine and the like and salts thereof can be mentioned. It is also possible to use two or more kinds selected from these. Preferred are substituted or unsubstituted aliphatic tertiary amines such as diethanolamine, triethanolamine, triisopropanolamine, diphenhydramine and the like, and more preferred is, for example, aliphatic tertiary amine substituted by a group having a hydroxyl group such as triisopropanolamine, triethanolamine and the like and most preferred is triisopropanolamine.

The "ionic liquid" in the present invention refers to a molten salt which can be formed by mixing a mast cell degranulation inhibitor having a carboxyl group with an organic amine and which is a liquid (viscous liquid) at room temperature. While many of the above-mentioned mast cell degranulation inhibitors are poorly soluble in organic solvents, their solubility in organic solvents can be enhanced, and their skin permeability can also be enhanced, by conversion into ionic liquids. To this end, an organic amine having high lipophilicity is preferably used to enhance the solubility of the above-mentioned mast cell degranulation inhibitors in organic solvents. Preferably, an organic amine having lipophilicity comparable to or higher than that of triisopropanolamine can be used.

While the amount of an organic amine to be blended with the above-mentioned mast cell degranulation inhibitor is not particularly limited, it is desirably not less than an equimolar amount so that a salt with the carboxyl group possessed by the mast cell degranulation inhibitor will be formed to afford an ionic liquid. Preferably, the amount of an organic amine to be blended is 0.5- to 25-fold, preferably 1-to 25-fold, more preferably 1- to 2-fold, molar amount relative to the mast cell degranulation inhibitor. In the present invention, the amount of an organic amine relative to a mast cell degranulation inhibitor is a ratio relative to 1 mol of the carboxyl group possessed by the mast cell degranulation inhibitor.

When the amount of the organic amine to be blended exceeds 4-fold molar amount, the transdermal permeability improving effect may reach a plateau, and further improving effect may not be achieved, which is undesirable from the economical aspect. Moreover, the amount of the organic amine to be blended is preferably 0.5- to 10-fold amount, more preferably 0.5- to 2-fold amount, in a molar ratio. At these molar ratios, the stability of, for example, tranilast to light irradiation is sufficiently improved.

Examples of the dosage form of the external preparation of the present invention include ointment, lotion, aerosol, plaster, aqueous adhesive skin patch and the like. The dosage form is not particularly limited as long as it is employed for an external preparation.

The external preparation of the present invention may contain additives generally known to and used by those of ordinary skill in the art, depending on the dosage form. Examples of the additive include formulation base, fatty acid or derivative thereof, alcohol, surfactant, suspending agent, thickener, solvent, solubilizing agent, inorganic particles, excipient, lubricant, stabilizer, wetting agent, buffering agent, pH control agent, colorant, flavor, binder, disintegrant, propellant and the like.

Examples of the above-mentioned formulation base include components of oily formulation base or hydrophobic formulation base, hydrophilic formulation base or components of hydrophilic formulation base, gel formulation base and the like. Examples of the above-mentioned component of oily formulation base or hydrophobic formulation base include natural rubber, rubbers such as isoprene rubber, polyisobutylene, styrene-isoprene-styrene block copolymer, styrene-butadiene-styrene block copolymer, styrene-ethylenebutylene-styrene block copolymer, (meth)acrylic acid alkyl ester (co)polymer, polyacrylate, methacrylic acid ester, polyisobutylene, polybutene, liquid polyisoprene and the like, oils such as petrolatum, cetanol, beeswax, white beeswax, lanolin, purified lanolin, liquid paraffin, paraffin wax, Plastibase containing liquid paraffin and polyethylene, silicone oil, triglyceride, squalene, microcrystalline wax, whale wax and the like, and the like.

Examples of the above-mentioned components of hydrophilic formulation base or water-permeated formulation base include hydrophilic fatty acid ester such as glycerol ester of saturated fatty acid and the like, aqueous polymer such as polyethylene glycol and the like, and the like. Examples of the above-mentioned components of gel base include carboxyvinyl polymer, starch acrylate, sodium polyacrylate, tragacanth, alginates, cellulose derivatives such as methylcellulose, carmellose, carmellose sodium, hydroxypropylcellulose, hydroxypropylmethylcellulose and the like, colloid clay composed of silicates such as bentonite, veegum and the like, aqueous basic substances such as carboxyvinyl polymer, polyvinyl alcohol, polyvinylpyrrolidone, alkali hydroxide, alkanolamine and the like, and the like.

Examples of the above-mentioned fatty acid and derivative thereof include higher fatty acids such as oleic acid, stearic acid and the like and salts thereof, esters of higher fatty acids such as caprylic acid, caproic acid, myristic acid, palmitic acid, stearic acid, oleic acid and the like and monovalent aliphatic alcohol (e.g., isopropyl myristate, isopropyl palmitate, isopropyl stearate, decyl oleate etc.), triglycerides such as triglyceride caprylate, triglyceride caproate, peanut oil, castor oil, cacao oil, hydrogenized oil (e.g., hardened castor oil etc.) and the like, fatty acid ester of polyvalent alcohol such as pentaerythritol fatty acid ester and the like, and the like. Examples of the ester of polyvalent carboxylic acid and alcohol include esters of polyvalent carboxylic acid such as adipic acid, sebacic acid and the like and monovalent aliphatic alcohol such as diethyl sebacate, ethyl adipate, diisopropyl adipate etc., and the like.

Examples of the above-mentioned alcohol include higher alcohols such as benzyl alcohol, lauryl alcohol, myristyl alcohol, cetyl alcohol, stearyl alcohol, cetostearyl alcohol, 2-octyldodecanol and the like, lower alcohols such as ethanol, isopropanol and the like, polyvalent alcohols such as ethylene glycol, glycerol, propylene glycol, 1,3-butylene alcohol and the like, and the like.

Examples of the above-mentioned surfactant include natural emulsifiers such as gum arabic, gelatin, tragacanth, lecithin, cholesterol and the like, anionic surfactants such as soap, alkylsodium sulfate and the like, sorbitan polyoxyethylene fatty acid esters such as monooleyl sorbitan polyoxyethylene and the like, glycerol fatty acid esters such as polyoxyethylenecastor oil derivative, polyoxyethylene hardened castor oil, glycerol monostearate, sorbitan monooleate and the like, sorbitan fatty acid esters such as sorbitan monostearate, sorbitan sesquioleate and the like, polyoxyethylene higher alcohol ethers such as polyoxyethylenecetyl ether and the like, nonionic surfactants such as polyoxyethylene alkylphenol, polyoxyethylene oxypropylene copolymers (e.g., pluronic etc.) and the like, cationic surfactants such as cetyl trimethylammonium chloride and the like, amphoteric surfactants and the like.

Examples of the above-mentioned suspending agent or thickener include polysaccharides such as gum arabic, tragacanth, pullulan, locust bean gum, tamarind gum, pectin, xanthan gum, guar gum, carageenan and the like, methylcellulose, carmellose, carmellose sodium, polyvinyl alcohol, polyvinylpyrrolidone, acrylic acid copolymer, carboxyvinyl polymer, colloidal, microcrystalline cellulose and the like.

Examples of the above-mentioned solvent include water, propylene glycol, butylene glycol, isopropanol, ethanol, glycerol, diethyl sebacate, isopropyl myristate, diisopropyl adipate, myristyl palmitate, stearyl stearate, myristyl myristate, seril lignocerate, lacceril cerolate, lacceril laccerate and the like.

Examples of the above-mentioned solubilizing agent include carmellose sodium, propylene glycol, polysorbate80, sodium benzoate, benzyl benzoate, urethane, monoethanolamine, diethanolamine, glycerol, sodium salicylate, diethylacetamide, sodium hydroxide, sodium carbonate, urea, N-hydroxyethyl lactam, monomethylacetamide, N-methyl-2-pyrrolidone and the like.

Examples of the above-mentioned inorganic particles include talc, silicic anhydride, calcium carbonate, magnesium carbonate, colloidalsilica, bentonite and the like.

Examples of the above-mentioned excipient include saccharides such as sucrose and the like, sugar alcohols such as mannitol and the like, starch derivatives such as dextrin and the like, cellulose derivatives such as crystalline cellulose and the like, inorganic substances such as calcium phosphate and the like, and the like. Examples of the above-mentioned lubricant include stearic acid metal salts such as calcium stearate, magnesium stearate and the like, lauryl sulfates such as magnesium lauryl sulfate and the like, starch derivatives recited as the above-mentioned excipient, and the like.

Examples of the above-mentioned stabilizer include preservative, antioxidant and the like. Examples of the above-mentioned preservative include parahydroxybenzoic acid esters such as methylparaben, propylparaben and the like, alcohols such as chlorobutanol, benzyl alcohol, phenylethyl alcohol and the like, thimerosal, acetic anhydride, sorbic acid and the like. Examples of the above-mentioned antioxidant include sodium bisulfite, L-ascorbic acid, sodium ascorbate, butylhydroxyanisole, butylhydroxytoluene, propyl gallate, tocopherol acetate, dl-α-tocopherol and the like.

Examples of the above-mentioned wetting agent include polyvalent alcohols such as glycerol, propylene glycol, butylene glycol, sorbitol and the like, and the like.

In addition to these additives, the above-mentioned buffering agent, pH control agent, colorant, flavor, binder, disintegrant and the like can also be added as necessary and, for example, peppermint oil, 1-menthol, camphor, thymol, tocopherol acetate, glycyrrhizinic acid, nonylic acid vanillylamide, capsicum extract and the like can also be added.

In addition to these additives, moreover, pharmaceutical products containing other drugs can also be added as long as the action and effect of the external preparation of the present invention are not prevented.

The additives exemplified above are appropriately selected depending on the dosage form of the external preparation of the present invention. The amounts thereof to be blended are also appropriately selected within the range generally used for each dosage form.

The dosage form of the external preparation of the present invention is not particularly limited as long as the preparation can be topically administered to the skin. For example, ointment, cream, gel, patch, tape, lotion, aerosol, plaster, adhesive preparation, liquid, liniment and the like can be mentioned. Of these, ointment, cream, gel, patch and tape are preferable, and ointment is preferable from among these since it can be appropriately applied to the affected part of dermatic diseases, irrespective of the size of the diseased part and without liquid dripping and the like. To prepare the above-mentioned ointment, a base is appropriately selected from the above-mentioned additives and employed.

The production method of the external preparation of the present invention is explained below. The external preparation of the present invention can be produced using a suitable method known to those of ordinary skill in the art, and the production method is not particularly limited. For example, the external preparation of the present invention can be obtained by adding a mast cell degranulation inhibitor having a carboxyl group to an organic amine and, for example, a suitable formulation base mentioned above, which is known to those of ordinary skill in the art, further adding other suitable additives also mentioned above, and mixing them. In addition, they can also be heated as necessary. In this way, a salt of the above-mentioned mast cell degranulation inhibitor and organic amine can be easily produced by bringing them into contact, for example, by preparing a composition containing them in the same system, particularly by kneading the composition and the like.

The external preparation of the present invention can be applied to an affected part of dermatic diseases by, for example, applying, coating, spraying and the like depending on the dosage form. The application dose of the above-mentioned external preparation to an affected part can be selected based on the content of the active ingredient and the like and, for example, the preparation can be applied once or more than once a day, where the frequency of application is not particularly limited.

The external preparation of the present invention explained above has improved transdermal permeability. When the photostability of the mast cell degranulation inhibitor, which is the main drug, is poor, it can be improved, and application as an external preparation becomes possible.

The present invention is explained in more detail in the following by referring to Examples, which are not to be construed as limitative. The present invention can be appropriately modified within the range compatible with the above-mentioned and below-mentioned gist and practiced. Such modifications are all encompassed in the technical scope of the present invention.

### Examples

### Example 1: Formation of salt of mast cell degranulation inhibitor and organic amine

Using tranilast and cromoglycic acid Na as mast cell degranulation inhibitors and triisopropanolamine as an organic amine, a 1:1 salt was formed (formation of ionic liquid) as in the following.

### (1) Formation of salt of tranilast and triisopropanolamine

Tranilast (0.50 g) and triisopropanolamine (0.29 g) were weighed, and the both compounds were melted by heating in a flask at 80°C for 30 min. The molten liquid remained viscous even at room temperature.

IR absorption of the carboxyl group of tranilast appears at a position of 1690 (1/cm⁻²) when measured by a KBr method. On the other hand, the IR absorption of the molten viscous liquid was measured to find that the absorption at 1690 (1/cm⁻²) corresponding to the carboxyl group had disappeared. In addition, as a new IR absorption, absorption at a position of 1585 (1/cm⁻²) had increased. IR of the obtained molten salt was measured using NaCl cell dissolved in chloroform.

From the IR results, it is shown that a salt of tranilast and triisopropanolamine was formed as a molten salt in the form of an ionic liquid.

### (2) Formation of salt of cromoglycic acid and triisopropanolamine

Sodium cromoglycate (10 g) was dissolved in about 200 ml of purified water at room temperature, adjusted to pH 2 with hydrochloric acid, and the precipitated crystals were collected by filtration. The obtained crystals were washed with purified water and dried under reduced pressure to give cromoglycic acid as a white bulk. This was pulverized to give a powder.

Cromoglycic acid and triisopropanolamine were weighed at a molar ratio of 1:5, and the both compounds were melted by heating in a flask at 80°C for 30 min. The IR of the obtained molten salt showed a similar behavior as the above-mentioned IR. The IR absorption shows the occurrence of an ionic liquid.

Excess triisopropanolamine was evaporated by further heating under reduced pressure to give a 1:1 salt.

### Example 2: Formation of salt of tranilast and various organic amine compounds and its solubility in ester compound

Test solutions containing tranilast were prepared according to the formulations shown in Table 1 (wt%, diethyl sebacate as a balance to set the whole to 100 wt%; in the following, the "balance" refers to wt% of each substance added to set the whole to 100 wt%), and the solubility of organic amine salt of tranilast in diethyl sebacate was examined. The organic amine compound added for salt formation was added at a ratio of 2 mol per 1 mol of tranilast. As comparison example 1, the solubility of tranilast in diethyl sebacate was examined.

**Table 1**

| | test solution 1 | test solution 2 | test solution 3 | test solution 4 | Comp. Ex. 1 |
|---|---|---|---|---|---|
| tranilast | 1 | 1 | 1 | 1 | 1 |
| monoethanolamine | 0.37 | | | | |
| diethanolamine | | 0.64 | | | |
| triethanolamine | | | 0.9 | | |
| triisopropanolamine | | | | 1.16 | |
| diethyl sebacate | balance | balance | balance | balance | balance |

| | | | | | |
|---|---|---|---|---|---|
| (numerical values are in wt%) | | | | | |

For evaluation of the solubility of tranilast in diethyl sebacate, the mixture was stirred at 80°C for 15 min, the solubility of tranilast in diethyl sebacate was evaluated. The results are shown in the following Table 2.

**Table 2**

| | solubility in diethyl sebacate (wt%) |
|---|---|
| test solution 1 | 0.5% |
| test solution 2 | 0.5% |
| test solution 3 | 0.7% |
| test solution 4 | 1.0% or above |
| Reference Example 1 | 0.3% |

| | |
|---|---|
| (Note) Tranilast was completely dissolved in test solution 4. | |

The results show that the solubility of tranilast was improved by forming an organic amine salt (formation of ionic liquid) by addition of an organic amine. Since tranilast was completely dissolved and blended in the solvent in the case of test solution 4 (triisopropanolamine), excess tranilast was further dissolved and possible addition up to a 1:1 molar ratio was suggested.

Transdermal permeability is generally higher for lipophilic substances. Thus, test solutions 1 to 4 that became soluble in oil such as diethyl sebacate are considered to have improved transdermal permeability.

Furthermore, from the experiment results, an amine salt of tranilast obtained by the addition of, from among organic amine compounds, an organic amine compound having higher lipophilicity than triisopropanolamine is considered to have further improved transdermal permeability.

### Example 3: Formation of 1:1 salt of tranilast and triisopropanolamine and its solubility of salt in various solvents

For preparation of test solutions (blending corresponding to 1 mol of triisopropanolamine), tranilast (1 wt%) and triisopropanolamine (0.56 wt%) were added, and glycerol, propylene glycol, butylene glycol, salicylic acid ethylene glycol, diethyl sebacate and isopropyl myristate were used as a balance to achieve 100 wt%, whereby each test solution was prepared.

For preparation of comparison control (without triisopropanolamine), tranilast (1 wt%) was added, and propylene glycol, salicylic acid ethylene glycol, diethyl sebacate and isopropyl myristate were used as a balance to achieve 100 wt%, whereby each test solution was prepared.

Using the test solutions and comparative controls prepared above, the solubility of tranilast in various solvents with or without triisopropanolamine was examined.

The results are shown in the following Table 3 (difference in solubility of tranilast with or without triisopropanolamine).

**Table 3**

| kind of solvent | 1:1 salt with triisopropanolamine | comparison control (tranilast alone) |
|---|---|---|
| glycerol | ○ | × |
| propylene glycol | ○ | × |
| butylene glycol | ○ | × |
| salicyl acid ethylene glycol | ○ | × |
| diethyl sebacate | ○ | × |
| isopropyl myristate | × | × |

| | | |
|---|---|---|
| (O: complete dissolution of tranilast, x: no dissolution of tranilast) | | |

From these results, the solubility of tranilast in each solvent was confirmed to increase by forming a 1:1 salt of tranilast by addition of triisopropanolamine (formation of ionic liquid).

### Example 4: photostability of tranilast (1:1 salt with triisopropanolamine)

A test solution 5 containing a salt of tranilast and triisopropanolamine (1:1) was prepared as in the following. In addition, a test solution free of triisopropanolamine was prepared as a comparison example (comparison example 2).

### (Preparation of test solution 5)

Tranilast (0.5% by weight, tranilast) and triisopropanolamine (0.3% by weight) and N-methyl-2-pyrrolidone (8% by weight) were mixed with heating, and propylene glycol (91.2% by weight) was added to the total 100% by weight. The mixture was stirred and mixed to give a test solution 5.

### (Preparation of comparison example 2)

Tranilast (0.5% by mass) and N-methyl-2-pyrrolidone (8% by mass) were mixed with heating, and propylene glycol (91% by mass) was added to the total 100% by mass. The mixture was stirred and mixed to give a comparison example 2.

The above-mentioned test solution 5 and comparison example 2 were placed under fluorescent light (about 3000 Lux) and the tranilast content of each test solution was measured by high performance liquid chromatography after 2 days from the light irradiation of the fluorescent light.

The test solution 5 and comparison example 2 were placed under direct sunlight, and the tranilast content of each test solution was measured by high performance liquid chromatography after 2 hr, 4 hr and 8 hr from the light irradiation of the direct sunlight.

The above-mentioned results are shown in Table 4 and Table 5.

**Table 4**

| | test solution 5 | comparison example 2 |
|---|---|---|
| 2 days after diffused light in the room | 96.0% | 92.0% |

| | | |
|---|---|---|
| (% value: tranilast content after time lapse relative to tranilast content (100%) at start) | | |

**Table 5**

| | test solution 5 | comparison example 2 |
|---|---|---|
| after direct sunlight for 2 hr | 82.3% | 62.7% |
| after direct sunlight for 4 hr | 80.1% | 57.2% |
| after direct sunlight for 8 hr | 71.3% | 51.2% |

| | | |
|---|---|---|
| (% value: tranilast content after time lapse relative to tranilast content (100%) at start) | | |

From the above results, in test solution 5 containing a salt with triisopropanolamine (1:1), a decrease in the tranilast content due to the direct sunlight irradiation was markedly suppressed as compared to comparison example 2 free of triisopropanolamine. That is, the stability of tranilast against light irradiation was found to be markedly improved by forming a salt with triisopropanolamine (formation of ionic liquid).

### Example 5: Formation of salt of tranilast and various organic amine compounds and its photostability

By mixing according to the composition (wt%) shown in Table 6, test solutions 6, 7 and 8 and comparison example 3 were prepared. The test solutions were subjected to the irradiation of diffused light in the room (about 700 Lux/h) for 0 hr, 4 hr, 48 hr and 96 hr, or direct sunlight for 0 hr, 2 hr, 4 hr and 8 hr. The tranilast concentration was measured and analyzed by high performance liquid chromatography in light shielding.

**Table 6**

| | test solution 6 | test solution 7 | test solution 8 | comparison example 3 |
|---|---|---|---|---|
| tranilast | 0.5 | 0.5 | 0.5 | 0.5 |
| triethanolamine | 4 (17.8-fold mol) | | | |
| diethanolamine | | 4 (25-fold mol) | | |
| diphenhydramine | | | 4 (10.4-fold mol) | |
| solubilizing agent (N-methyl-2-pyrrolidone) | 8 | 8 | 8 | 8 |
| propylene glycol | balance | balance | balance | balance |

| | | | | |
|---|---|---|---|---|
| (numerical values are in wt% in Table) | | | | |

The photostability test results are shown in Table 7 and Table 8.

**Table 7**

| | test solution 6 | test solution 7 | test solution 8 | comparison example 3 |
|---|---|---|---|---|
| after 4 hr of fluorescent light | 101.5% | 96.0% | 100.1% | 99.6% |
| after 48 hr of fluorescent light | 101.7% | 96.4% | 99.9% | 94.9% |
| after 96 hr of fluorescent light | 99.9% | 94.6% | 99.9% | 89.4% |

| | | | | |
|---|---|---|---|---|
| (numerical values in Table show tranilast concentration relative to initial value (proportion relative to 0 hr)) | | | | |

**Table 8**

| | test solution 6 | test solution 7 | test solution 8 | comparison example 3 |
|---|---|---|---|---|
| after direct sunlight for 2 hr | 95.5% | 90.4% | 95.3% | 73.2% |
| after direct sunlight for 4 hr | 89.5% | 86.7% | 90.6% | 62.3% |
| after direct sunlight for 8 hr | 87.4% | 83.1% | 85.3% | 57.3% |

| | | | | |
|---|---|---|---|---|
| (numerical values in Table show tranilast concentration relative to initial value (proportion relative to 0 hr)) | | | | |

From the above results, it has been clarified that the photostability of tranilast against light irradiation can be improved by forming a salt with an organic amine.

### Example 6: Preparation of ointment containing triisopropanolamine salt (1:1) of mast cell degranulation inhibitor tranilast

Each ointment was prepared according to the formulation shown below.

### (Preparation of ointment 1)

Ointment 1 containing a salt of tranilast with triisopropanolamine (1:1) was prepared according to the formulation shown in Table 9. Specifically, tranilast, triisopropanolamine, light anhydrous silicic acid, polyvinylpyrrolidone and propylene glycol were mixed, dissolved by heating at 80°C, blended with Plastibase and preservative (methyl paraoxybenzoate) to give ointment 1.

### (Preparation of ointment 2)

Ointment 2 containing a salt of tranilast with triisopropanolamine (1:1) was prepared according to the formulation shown in Table 9. Specifically, tranilast, triisopropanolamine, light anhydrous silicic acid, polyvinylpyrrolidone and butylene glycol were mixed, dissolved by heating at 80°C, blended with Plastibase and preservative (methyl paraoxybenzoate) to give ointment 2.

### (Preparation of comparison control)

Ointment 3 containing tranilast but free of an amine compound was prepared according to the formulation (wt%) shown in Table 9. Specifically, tranilast, light anhydrous silicic acid, polyvinylpyrrolidone and propylene glycol were mixed, dissolved by heating at 80°C, blended with Plastibase and preservative (methyl paraoxybenzoate) to give a comparison control.

**Table 9**

| | ointment 1 | ointment 2 | comparison control |
|---|---|---|---|
| tranilast | 1 | 1 | 1 |
| triisopropanolamine | 0.56 | 0.56 | |
| light anhydrous silicic acid | 1 | 1 | 1 |
| polyvinylpyrrolidone | 3 | 3 | 3 |
| propylene glycol | 30 | | |
| butylene glycol | | 30 | 30 |
| methyl paraoxybenzoate | 0.16 | 0.16 | 0.16 |
| Plastibase | balance | balance | balance |

| | | | |
|---|---|---|---|
| (numerical values are in wt%) | | | |

### Experimental Example 1: Transdermal permeability test of ointment containing salt of tranilast with triisopropanolamine (1:1)

Ointments 1, 2 and comparison control were applied by 0.02 g to the arms of two test subjects A, B in a circular area (diameter 1.5 cm). After 12 hr, the plaster on the skin surface was wiped and a tape was adhered to the skin surface to cover the part other than the above-mentioned circular area applied with the ointment. Then, an adhesive tape was adhered to and detached from the above-mentioned circular area alone to provide samples. The first two samples were removed since tranilast remaining on the skin surface might be incorporated.

Using five adhesive sheets, sampling was performed 5 times and the 5 sheets were collectively used as one sample (column 1). This operation was repeated for 5 more columns, whereby samples for columns 1-6 were obtained for each of two test subjects. Tranilast was extracted from each samples with methanol, and the amount of tranilast was measured by high performance liquid chromatography, and the intradermal transfer amount of tranilast was calculated.

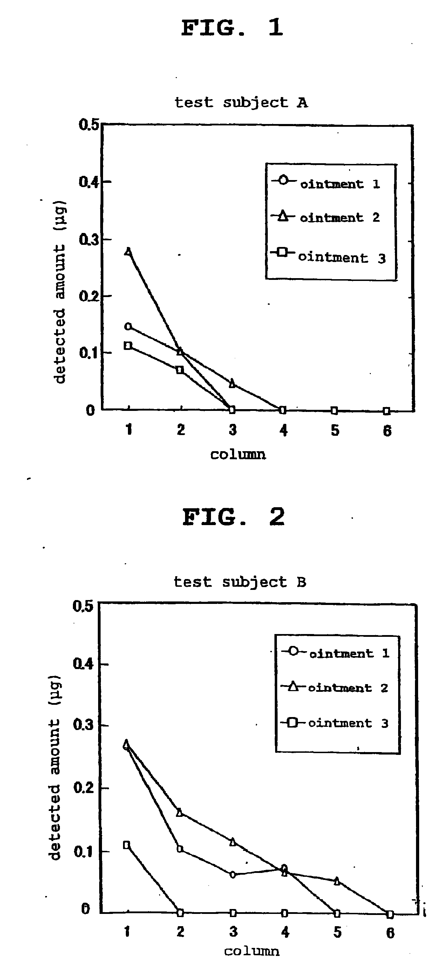

The results are shown in Figs. 1, 2 and Table 10 (intradermal transfer amount of tranilast).

**Table 10**

| | ointment 1 | ointment 2 | comparison control |
|---|---|---|---|
| test subject A | 0.247 | 0.429 | 0.177 |
| test subject B | 0.505 | 0.667 | 0.108 |

| | | | |
|---|---|---|---|
| (unit: µg) | | | |

In Figs. 1, 2, columns 1-6 each show sampling sites. Column 1 shows sampling on the skin surface, and the numbers toward column 5 indicate that sampling was performed at increasingly lower layers of the skin.

From these results, it has been clarified as shown in Figs. 1, 2, and Table 10 that ointments 1, 2, which are external preparations containing a salt of a mast cell degranulation inhibitor tranilast, and triisopropanolamine at 1:1, have remarkably high transdermal permeability as compared to comparison control without addition of triisopropanolamine.

### Industrial Applicability

The external preparation of the present invention shows superior skin permeability of a mast cell degranulation inhibitor having a carboxyl group, and improved photostability of the mast cell degranulation inhibitor. Therefore, by formation of an ionic liquid, the preparation enables a new application as an external preparation of a mast cell degranulation inhibitor which has heretofore been used solely by oral administration as a therapeutic agent for allergic dermatitis. By administration of the external preparation of the present invention directly to an affected part of dermatic diseases, liver metabolism and systemic exposure that are problems in oral administration can be avoided, and side effects of the mast cell degranulation inhibitor can be reduced.

In addition, since the external preparation of the present invention shows improved photostability, an external preparation effective for the affected part of dermatic diseases, such as face and limbs easily exposed to sun light, can be provided. Therefore, the external preparation of the present invention can sufficiently exert the action effect of a mast cell degranulation inhibitor.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2005-306520 filed in Japan (filing date: October 21, 2005), the contents of which are incorporated in full herein by this reference.

## Claims

1. An external preparation comprising a salt of a mast cell degranulation inhibitor having a carboxyl group with an organic amine.

2. The external preparation of claim 1, wherein the amount of the organic amine blended relative to the mast cell degranulation inhibitor is 0.5- to 10-fold amount in a molar ratio.

3. The external preparation of claim 1 or 2, wherein the organic amine is selected from triisopropanolamine, triethanolamine, diethanolamine and diphenhydramine.

4. The external preparation of any of claims 1 to 3, wherein the mast cell degranulation inhibitor is tranilast or cromoglycic acid.

5. The external preparation of claim 1 or 2, wherein the mast cell degranulation inhibitor is tranilast and the organic amine is triisopropanolamine.

6. A method of producing an external preparation with improved photostability, which comprises adding an organic amine to cause formation of a salt, so as to improve photostability of a mast cell degranulation inhibitor having a carboxyl group.

7. The production method of claim 6, wherein the amount of the organic amine blended relative to the mast cell degranulation inhibitor is 0.5- to 10-fold amount in a molar ratio.

8. The production method of claim 6 or 7, wherein the organic amine is selected from triisopropanolamine, triethanolamine, diethanolamine and diphenhydramine.

9. The production method of any of claims 6 to 8, wherein the mast cell degranulation inhibitor is tranilast or cromoglycic acid.

10. The production method of claim 6 or 7, wherein the mast cell degranulation inhibitor is tranilast and the organic amine is triisopropanolamine.

11. A method of improving photostability of a mast cell degranulation inhibitor having a carboxyl group, which comprises blending an organic amine with the mast cell degranulation inhibitor to cause formation of a salt.

12. The method of claim 11, wherein the amount of the organic amine blended relative to the mast cell degranulation inhibitor is 0.5- to 10-fold amount in a molar ratio.
